# EUROPEAN PATENT SPECIFICATION

(11) **EP 2 657 685 B1**
(45) Date of publication and mention of the grant of the patent: **22.05.2019**
(21) Application number: 12425079.6
(22) Date of filing: 27.04.2012
(51) Int. Cl.: G01N 21/3504, F28F 3/12, F28F 3/04, G01F 5/00, G01N 33/00, F28F 7/02, G01N 21/84, G01N 21/85, G01N 1/22

(54) **Gas analysis and control system, in particular for the detection of the Lower Explosion Limit (LEL) value of a gas or gaseous mixture**
Gasanalyse- und -steuerungssystem, insbesondere zur Detektion des Werts der untersten Explosionsgrenze (UEG) eines Gases oder einer gasförmigen Mischung
Système de commande et d'analyse de gaz, en particulier pour la détection de la valeur limite inférieure d'explosivité (LEL) d'un gaz ou mélange gazeux

(43) Date of publication of application: 30.10.2013
(73) Proprietor: BOBST ITALIA S.P.A., 29121 Piacenza (PC) (IT)
(72) Inventor: Robotti, Maurizio, 15010 Casale Monferrato (Alessandria) (IT)
(74) Representative: Hasler, David

(56) References cited:
- EP-A2- 1 873 510
- DE-A1-102008 041 828
- US-A- 5 060 572
- US-A1- 2006 202 123
- US-A1- 2010 228 688
- BOSSART C J: "Monitoring and control of combustible gas concentrations below the lower explosive limit", ANALYSIS INSTRUMENTATION, VOL.12 ISA PITTSBURGH, PA, USA, 1974, pages 71-79, XP009162757,

## Description

### Technical Field

The present invention relates to a gas analysis and control system, in particular for the detection of the Lower Explosion Limit (LEL) value of a gas or a gaseous mixture, in particular suitable for use in an integrated safety system for the analysis of gases that arose in printing processes, such as rotogravure printing groups.

### Background Art

The safety systems for the control and analysis of the gases derived from printing processes are well known in the printing industries. In particular these systems can be used for rotogravure printing presses in which a gravure printing roller applies ink or the like onto a film or web material to be printed, such as a packaging material.

After the film has been printed, there is a drying phase of the solvent used in the printing process. The drying causes the evaporation of the solvent, which passes in the gaseous state and has to be properly treated before its release into the environment. The gaseous mixture of air and solvent is transported through ducts and dryers to an incinerator which burns the solvent vapors contained in the mixture.

Various safety regulations are applied on rotogravure printing unit and those rules also cover the management of gases transported inside the ducts and the hoods of the printing unit.

One of the parameters of the regulation is the respect of a percentage value called LEL (Lower Explosion Limit) of the gaseous mixture air-solvent. A LEL level equal to 100% indicates a percentage of solvent into the gaseous mixture that leads to its detonation.

Therefore it's necessary, in applications like a rotogravure printing unit, a security system for the detection of the LEL value.

US 2010/0228688 A1 describes a chemical composition analyzer operable to optically determine and report the chemical composition of a gas within a gas collection and transmission infrastructure, comprising several features of the preamble of claim 1.

US 2006/0202123 A1 discloses a method for measuring the quantity of chemical species contained in a high temperature gas and especially the quantity of CO and CO₂ contained in a gas output by a metal treatment furnace.

DE 10 2008 041 828 A1 describes a heat exchanger for gas chromatography equipment, having cooling elements arranged individually and thermally spaced from each other along flow direction of one of fluid and thermally coupled with counter flow heat exchanger body.

US 5060572 A discloses a continuous printed web drier for use in rotary web offset printing presses and a method for operating the continuous drier, in which the press has actuators for control of heating, intake gas flow and exhaust gas flow.

EP 1873510 A2 discloses a method for analyzing a fluid in a fluid flow or tank and a device for implementing the method. The device comprises a measuring chamber in which a gaseous sample of the fluid is introduced.

Another known system is described in the publication "Monitoring and control of combustible gas concentrations below the lower explosive limit" (BOSSART C J), Analysis Instrumentation, Vol. 12 ISA Pittsburgh, PA, USA.

### Disclosure of invention

The purpose of the present invention is therefore to realize a gas analysis and control system for the detection of the Lower Explosion Limit (LEL) value of a gas or a gaseous mixture, in particular suitable for printing processes, in which said detection is carried out with a redundant control on the gaseous mixture flow rate, thereby respecting high level of security.

This purpose is achieved by the present invention by means of a gas analysis and control system for the detection of the Lower Explosion Limit (LEL) value of a gas or a gaseous mixture according to the combination of features of claim 1.

Further advantageous features of the gas analysis and control system according to the present invention have been specified in the dependent claims.

Advantageously, a double flow rate measurement, one upstream and one downstream of means for sensing of the LEL value, allows verifying any interruptions on connecting ducts and pipes with the consequent suction of air from the environment where, in fact, the solvent is not there. The present system is a redundant system and by its nature respects regulations for security devices and systems.

Preferably, the double flow rate measurement is carried out by means of two calibrated orifice diaphragms for measuring flow rate indirectly through the reading of the gaseous mixture pressure value.

Further characteristics and advantages of the present invention will be more apparent from the following description of a preferred embodiment thereof, illustrated only by way of non limitative example in the enclosed figures and layouts.

### Brief description of figures

Fig. 1 shows a functional layout of a gas analysis and control system for the detection of the Lower Explosion Limit (LEL) value of a gas or a gaseous mixture according to an exemplary embodiment of the present invention and its position within a rotogravure printing group.
Fig. 2 shows an isometric view of a tapping group provided in the present system and comprising an heat exchanger-meter group provided with an heat exchanger-meter plate.
Fig. 3A shows a longitudinal section view of the heat exchanger-meter plate comprising a calibrated orifice diaphragm.
Fig. 3B shows a top view of the heat exchanger-meter plate comprising two gas passage channels.
Fig. 3C shows a bottom view of the heat exchanger-meter plate comprising an heat exchange finning.

### Best Mode for Carrying Out the Invention

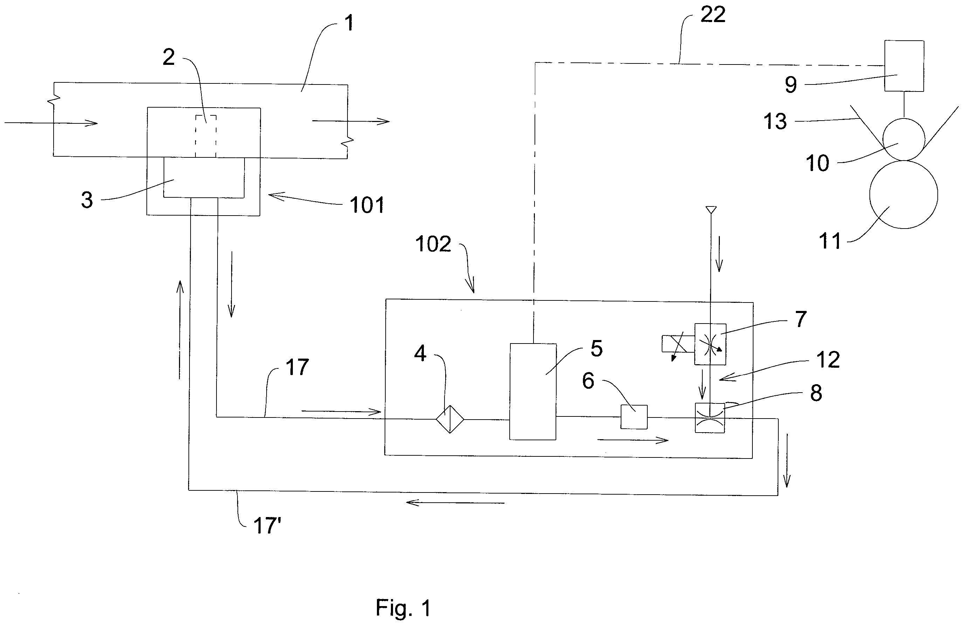

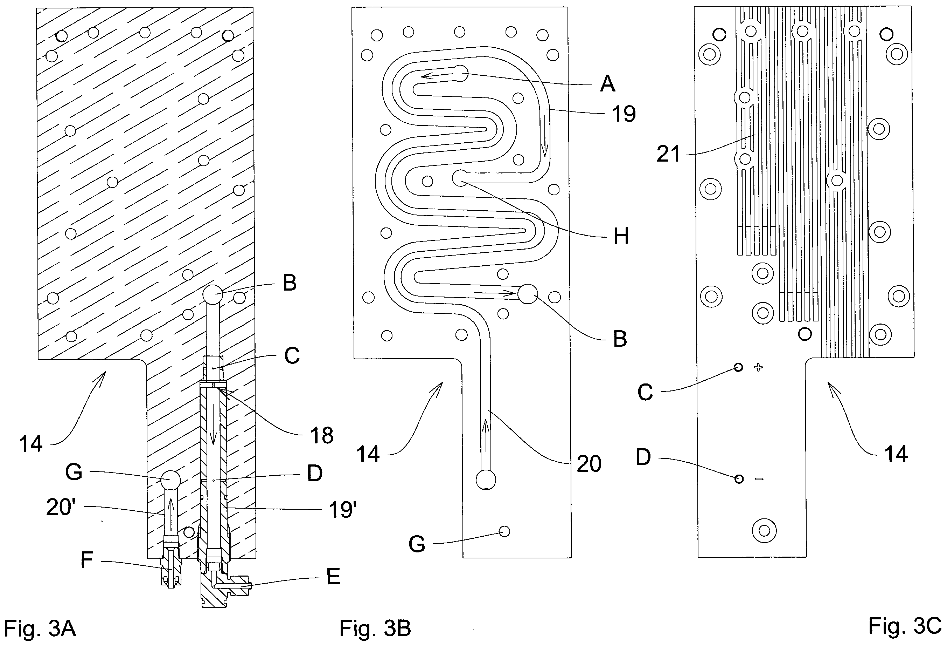

With reference to the layout represented in Fig. 1, the gas analysis and control system for the detection of the Lower Explosion Limit (LEL) value of a gas or a gaseous mixture, in accordance with the invention, comprises a tapping group 101 and a group 102 of measurement which are interfaced with a gas or gaseous mixture evacuation duct 1, with a pneumatic system 12 and, via an electric connection 22, with an actuation system 9 of a pressure roller 10 which presses a film 13 on a printing cylinder 11 of a printing unit, in particular a rotogravure printing group. The present system is provided with a first gas inlet line 17, for transferring gas from the tapping group 101 to the measurement group 102, and a second gas outlet line 17', for returning gas from the measurement group 102 to the tapping group 101 and therefore to the evacuation duct 1. The tapping group 101 comprises a suction filter 2 and a heat exchanger-meter group 3. The pneumatic system 12 is able to take air from external environment and send it to a proportional valve 7 of the measurement group 102. Said group of measurement 102 further comprises a filter 4, an infrared (IR) gas analyzer 5, a flow rate sensor 6 and an ejector 8 positioned in said first line 17. The ejector 8, using Venturi principle with incoming air from the pneumatic system 12, creates a flow rate sucking the gas present in the evacuation duct 1 through the suction filter 2. Suction is also facilitated by the direction of motion of the gas in the evacuation duct 1, symbolized by the arrows. The gas drawn off, that is a gaseous mixture of solvent and air, passes through the heat exchanger-meter group 3, which allows an indirect measure of flow rate through the reading of the pressure, see Fig. 3A, by means of a first calibrated orifice diaphragm 18, provided in a channel 19'. The heat exchanger-meter group 3 allows also to reduce the sucked gas temperature, essential condition to have a good measure by the IR gas analyzer 5. Subsequently, the gas from the line 17 comes to a filter 4 that cleans the gas from waste that may damage the IR gas analyzer 5. After the filter 4, gas is analyzed by the IR gas analyzer 5; if the LEL percentage value is greater than a predetermined level, the safety condition of the printing unit is realized, that is the analyzer sends an electrical signal to the system 9 of the pressure roller 10, which departs from the printing cylinder 11; consequently the printing process is stopped. In the case where the analyzed LEL percentage is lower than the set level, the printing process has the consent to continue. After the IR gas analyzer 5, the gas enters into the flow rate sensor 6 consisted of a calibrated orifice diaphragm, similar to the calibrated diaphragm 18 of Fig. 3A. Said calibrated orifice diaphragm allows to calculate the flow rate value in an indirect way through the reading of the pressure value. Subsequently, the gas enters in the ejector 8 which receives a quantity of air regulated by the proportional valve 7. In the ejector 8, taking advantage of the Venturi principle, the gas mixed with the air of the pneumatic system exits and returns, via the line 17', to the heat exchanger-meter group 3. The proportional valve 7 is adjusted according to the pressure difference between the downstream reading at the flow rate sensor 6, and the upstream reading at the heat exchanger-meter group 3. From the reading of the pressure difference, therefore of the flow rate difference, the present system, by means of a computer system, determines if there is a loss in the path between the two points of reading, and then it puts in safety condition the rotogravure printing unit, or if the level of cleaning of the filter 4 has reached a predetermined limit.

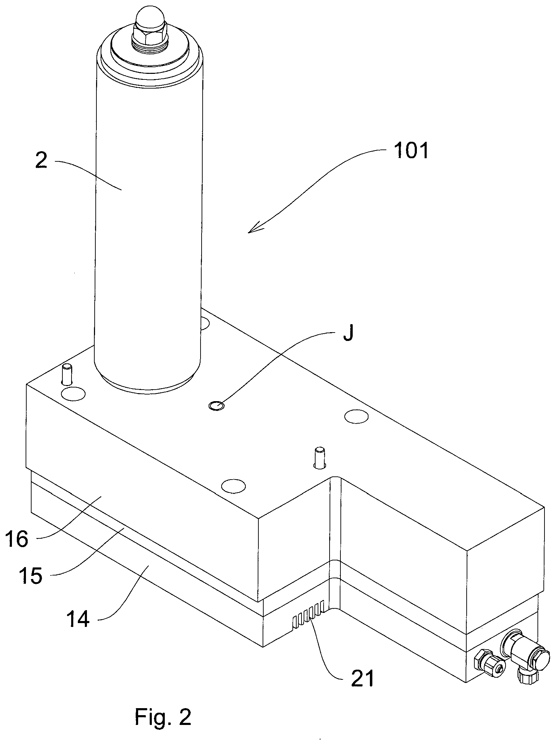

The gas that returns to the heat exchanger-meter group 3, out from the ejector 8 and via the line 17', passes through a heat exchange plate 14, see Fig. 2, integrated in the group 3 and subsequently it can returns to the evacuation duct 1. The heat exchange plate 14 embedded in the element 3 cools the gas drawn off at the beginning of the cycle described. The tapping group 101 represented in Fig. 2 includes the filter 2, an insulator layer 16, a plate 15 and the heat exchanger-meter plate 14. The heat exchanger-meter plate 14, see also Fig. 3A, 3B, 3C, comprises a single aluminum plate in which are formed: on the top surface two paths or channels 19 and 20 for the gaseous mixture, on the bottom surface a heat exchange finning 21 and inside, in the thickness of the plate, two channels 19' and 20' for the passage of the gas. The channel 20' is connected to the path 20 and the channel 19' with calibrated orifice diaphragm 18 is connected to the path 19 and is obtained from an unique block. The arrows showed in Fig. 3A and 3B represent the direction of the gaseous mixture along the heat exchange plate 14.

With reference to Figures 1, 2 and 3A, 3B, 3C above described, during the inlet phase, the air-solvent gaseous mixture is sucked from the evacuation duct 1 and enters the suction filter 2, by means of the Venturi ejector 8. The gaseous flow reaches the point A of the path 19 formed on the surface of the plate 14 and it runs to the point B. From point B, the gaseous mixture passes through the calibrated orifice diaphragm 18, points C and D, exits from the plate 14 at point E, continues its path via the line 17 and reaches the measurement group 102, according to the layout of Fig. 1. In the outlet and return phase, the gaseous mixture leaves the measurement group 102, reaches the point F of the plate 14, via the line 17', and it proceeds up to the point G of the channel 20'. From the point G, the gaseous mixture flows through the outlet path 20 formed on the surface of the plate 14 arriving at point H. From the point H the gaseous mixture returns to the evacuation duct 1 through the hole J visible in Fig. 2. The gaseous mixture in the return line 17' is mixed with air from the pumping system 12 and returns to the tapping group 101 and therefore to the duct 1 upstream of the inlet of the line 17. Therefore said gaseous mixture can be eventually reintroduced in the tapping group 101 and therefore in the line 17. The two paths or channels 19 and 20 described above are formed by fastening of the heat exchanger-meter plate 14 with the plate 15. The insulating material layer 16 thermally insulates the plate 15 from the evacuation duct 1 at high temperature. Through said two paths 19 and 20 formed on the surface of the plate 14, the gas drawn off in the inlet phase is cooled by heat exchange due to the difference between its temperature and the outlet gas temperature. In addition, the gas drawn off is cooled by the heat transmission improved through the finning 21 of the heat exchanger-meter plate 14. The decrease of the temperature difference between the flow rate detected upstream of the gas analyzer 5, that is the reading in the tapping group 101, and the flow rate detected downstream of the gas analyzer 5, that is the reading in the sensor 6, improves the accuracy and reliability of the entire gas analysis and control system according to the present invention. Advantageously, said readings of the gas flow rate are obtained from the pressure readings by means of calibrated orifice diaphragms: a diaphragm in the heat exchanger-meter group 3 of the tapping group 101 and a diaphragm in the flow rate sensor 6. The present system can be used advantageously in printing groups, in particular for rotogravure printing groups, and has the function of gas analysis with redundant control on the flow rate of the gaseous mixture.

## Claims

1. Gas analysis and control system for the detection of the Lower Explosion Limit value of a gas or a gaseous mixture, in particular suitable for gases that arose in printing processes, comprising: a gas duct (1), a first inlet line (17) to draw off a gas or a gaseous mixture from the gas duct (1), a tapping group (101) interfaced with said gas duct (1) and with said first inlet line (17), a measurement group (102) positioned in said first line (17) downstream of said tapping group (101), and a second return line (17') positioned downstream of said measurement group (102) and able to return the gas or gaseous mixture from said measurement group (102) to said gas duct (1), said tapping group (101) comprising first means (3, 18) for detecting a first gas or gaseous mixture flow rate, and said measurement group (102) comprising an infrared gas analyzer (5) for detecting the Lower Explosion Limit value of the gas or gaseous mixture and second means (6) for detecting a second gas or gaseous mixture flow rate, **characterized in that** said measurement group (102) further comprises means (7, 8, 12) for supplying air downstream of said second means (6) and for mixing air with said gas or gaseous mixture according to the pressure difference between said first gas or gaseous mixture flow rate detection and said second gas or gaseous mixture flow rate detection, and **in that** said first means (3, 18) for detecting a first gas or gaseous mixture flow rate of said tapping group (101) comprises a heat exchanger-meter group (3) for measuring said first gas or gaseous mixture flow rate and for cooling the gas or gaseous mixture.

2. Gas analysis and control system according to claim 1, **characterized in that** said means (7, 8, 12) for supplying and mixing air comprises a pneumatic system (12) and a Venturi ejector (8), said measurement group (102) is interfaced with the pneumatic system (12) which is arranged to send air to the Venturi ejector (8) positioned on said first line (17) and able to create a flow rate sucking the gas or gas mixture present in said gas duct (1) through the tapping group (101).

3. Gas analysis and control system according to claim 1, **characterized in that** said heat exchanger-meter group (3) is provided with a calibrated orifice diaphragm (18) for measuring said first gas or gaseous mixture flow rate through the reading of the gas or gaseous mixture pressure value.

4. Gas analysis and control system according to claim 3, **characterized in that** said heat exchanger-meter group (3) comprises at least an heat exchanger-meter plate (14) realized in a single body and incorporating said calibrated orifice diaphragm (18).

5. Gas analysis and control system according to claim 4, **characterized in that** said heat exchanger-meter plate (14) comprises a first inlet channel (19') connected to said first line (17) and comprising said calibrated orifice diaphragm (18), and a second return channel (20') connected to said return line (17') and connected to said gas duct (1).

6. Gas analysis and control system according to claim 5, **characterized in that** said plate (14) comprises on one side first and second gas paths (19, 20) and on the other side an heat exchange finning (21), said tapping group (101) further comprises a cover plate (15) positioned on the side of said first and second gas paths (19, 20).

7. Gas analysis and control system according to claim 6, **characterized in that** said tapping group (101) comprises an insulating material layer (16) positioned on said cover plate (15) and able to thermally insulate the cover plate (15) from the gas duct (1) at high temperature.

8. Gas analysis and control system according to claim 4, **characterized in that** said plate (14) comprises a suction filter (2) connected to said gas duct (1).

9. Gas analysis and control system according to claim 1, **characterized in that** said second means (6) for detecting a second gas or gaseous mixture flow rate comprise a calibrated diaphragm positioned downstream of said infrared gas analyzer (5) of the measurement group (102).

10. Gas analysis and control system according to claim 1, **characterized in that** said means (7, 8, 12) for supplying and mixing air comprises a proportional valve (7) adjustable according to the pressure difference between said second gas or gaseous mixture flow rate reading by said second means (6) located in the measurement group (102) and said first gas or gaseous mixture flow rate reading by said first means (3, 18) located in the tapping group (101).

11. Gas analysis and control system according to claim 1, **characterized in that** said infrared gas analyzer (5) is electrically connectable to a printing roller actuation system (9) of a printing group.

## Patentansprüche

1. Gasanalyse- und -steuersystem zur Detektion des unteren Explosionsgrenzwerts eines Gases oder eines Gasgemischs, insbesondere geeignet für Gase, die bei Druckprozessen entstanden sind, umfassend: ein Gasrohr (1), eine erste Gaseinlassleitung (17), um ein Gas oder ein Gasgemisch aus dem Gasrohr (1) abzuziehen, eine Anzapfgruppe (101), die eine Schnittstelle mit dem Gasrohr (1) und mit der ersten Gasleitung (17) bildet, eine Messgruppe (102), die in der ersten Leitung (17) stromabwärts von der Anzapfgruppe (101) positioniert ist, und eine zweite Rückführleitung (17'), die stromabwärts von der Messgruppe (102) positioniert ist und in der Lage ist, das Gas oder Gasgemisch aus der Messgruppe (102) zum Gasrohr (1) zurückzuführen, wobei die Anzapfgruppe (101) erste Mittel (3, 18) zur Detektion einer ersten Gas- oder Gasgemischdurchflussrate umfasst, und die Messgruppe (102) einen Infrarot-Gasanalysator (5) zur Detektion des unteren Explosionsgrenzwerts des Gases oder Gasgemischs umfasst, und zweite Mittel (6) zur Detektion einer zweiten Gas- oder Gasgemischdurchflussrate, **dadurch gekennzeichnet, dass** die Messgruppe (102) weiter Mittel (7, 8, 12) zur Zufuhr von Luft stromabwärts von den zweiten Mitteln (6) und zum Mischen von Luft mit dem Gas oder Gasgemisch gemäß der Druckdifferenz zwischen der ersten Gas- oder Gasgemisch-Durchflussratendetektion und der zweiten Gas- oder Gasgemisch-Durchflussratendetektion umfasst, und dadurch, dass die ersten Mittel (3, 18) zur Detektion einer ersten Gas- oder Gasgemischdurchflussrate der Anzapfgruppe (101) eine Wärmetauscher-Messgerätgruppe (3) zur Messung der ersten Gas- oder Gasgemischdurchflussrate und zur Kühlung des Gases oder Gasgemischs umfassen.

2. Gasanalyse- und -steuersystem nach Anspruch 1, **dadurch gekennzeichnet, dass** die Mittel (7, 8, 12) zur Zufuhr und zum Mischen von Luft ein pneumatisches System (12) und einen Venturi-Ejektor (8) umfassen, wobei die Messgruppe (102) eine Schnittstelle mit dem pneumatischen System (12) bildet, das eingerichtet ist, Luft an den Venturi-Ejektor (8) zu schicken, der auf der ersten Leitung (17) positioniert ist und in der Lage ist, eine Durchflussrate zu erzeugen, die das Gas oder Gasgemisch, das in dem Gasrohr (1) vorhanden ist, durch die Anzapfgruppe (101) ansaugt.

3. Gasanalyse- und -steuersystem nach Anspruch 1, **dadurch gekennzeichnet, dass** die Wärmetauscher-Messgerätgruppe (3) mit einem kalibrierten Öffnungsdiaphragma (18) zur Messung der ersten Gas- oder Gasgemischdurchflussrate durch Ablesen des Gas- oder Gasgemisch-Druckwerts versehen ist.

4. Gasanalyse- und -steuersystem nach Anspruch 3, **dadurch gekennzeichnet, dass** die Wärmetauscher-Messgerätgruppe (3) mindestens eine Wärmetauscher-Messgerätplatte (14) umfasst, die in einem einzelnen Körper ausgeführt ist und das kalibrierte Öffnungsdiaphragma (18) einschließt.

5. Gasanalyse- und -steuersystem nach Anspruch 4, **dadurch gekennzeichnet, dass** die Wärmetauscher-Messgerätplatte (14) einen ersten Einlasskanal (19'), der mit der ersten Leitung (17) verbunden ist und das kalibrierte Öffnungsdiaphragma (18) umfasst, und einen zweiten Rückführkanal (20'), der mit der Rückführleitung (17') verbunden ist und mit dem Gasrohr (1) verbunden ist, umfasst.

6. Gasanalyse- und -steuersystem nach Anspruch 5, **dadurch gekennzeichnet, dass** die Platte (14) auf einer Seite einen ersten und zweiten Gasweg (19, 20) und auf der anderen Seite Wärmetauscherlamellen (21) umfasst, wobei die Anzapfgruppe (101) weiter eine Deckplatte (15) umfasst, die auf der Seite des ersten und zweiten Gaswegs (19, 20) positioniert ist.

7. Gasanalyse- und -steuersystem nach Anspruch 6, **dadurch gekennzeichnet, dass** die Anzapfgruppe (101) eine Isoliermaterialschicht (16) umfasst, die auf der Deckplatte (15) positioniert ist und in der Lage ist, die Deckplatte (15) gegen das Gasrohr (1) bei hoher Temperatur thermisch zu isolieren.

8. Gasanalyse- und -steuersystem nach Anspruch 4, **dadurch gekennzeichnet, dass** die Platte (14) einen Saugfilter (2) umfasst, der mit dem Gasrohr (1) verbunden ist.

9. Gasanalyse- und -steuersystem nach Anspruch 1, **dadurch gekennzeichnet, dass** die zweiten Mittel (6) zur Detektion einer zweiten Gas- oder Gasgemischdurchflussrate ein kalibriertes Diaphragma umfassen, das stromabwärts von dem Infrarot-Gasanalysator (5) der Messgruppe (102) positioniert ist.

10. Gasanalyse- und -steuersystem nach Anspruch 1, **dadurch gekennzeichnet, dass** die Mittel (7, 8, 12) zur Zufuhr und zum Mischen von Luft ein Proportionalventil (7) umfassen, das gemäß der Druckdifferenz zwischen der Ablesung der zweiten Gas- oder Gasgemischdurchflussrate durch die zweiten Mittel (6), die in der Messgruppe (102) angeordnet sind, und der Ablesung der ersten Gas- oder Gasgemischdurchflussrate durch die ersten Mittel (3, 18), die in der Anzapfgruppe (101) angeordnet sind, einstellbar ist.

11. Gasanalyse- und -steuersystem nach Anspruch 1, **dadurch gekennzeichnet, dass** der Infrarot-Gasanalysator (5) elektrisch mit einem Druckwalzen-Betätigungssystem (9) einer Druckgruppe verbunden werden kann.

## Revendications

1. Système de commande et d'analyse de gaz pour la détection de la valeur limite inférieure d'explosivité d'un gaz ou d'un mélange gazeux, en particulier adapté à des gaz qui sont apparus dans des processus d'impression, comprenant : un conduit de gaz (1), une première ligne d'entrée (17) pour aspirer un gaz ou un mélange gazeux du conduit de gaz (1), un groupe de prélèvement (101) relié audit conduit de gaz (1) et à ladite première ligne d'entrée (17),
un groupe de mesure (102) positionné dans ladite première ligne (17) en aval dudit groupe de prélèvement (101), et une seconde ligne de retour (17') positionnée en aval dudit groupe de mesure (102) et apte à faire retourner le gaz ou le mélange gazeux dudit groupe de mesure (102) audit conduit de gaz (1), ledit groupe de prélèvement (101) comprenant un premier moyen (3, 18) pour la détection d'un premier débit de gaz ou de mélange gazeux,
et ledit groupe de mesure (102) comprenant un analyseur de gaz à infrarouge (5) pour la détection de la valeur limite inférieure d'explosivité du gaz ou du mélange gazeux et un second moyen (6) pour la détection d'un second débit de gaz ou de mélange gazeux, **caractérisé en ce que** ledit groupe de mesure (102) comprend en outre un moyen (7, 8, 12) pour l'alimentation en air en aval dudit second moyen (6) et pour le mélange d'air avec ledit gaz ou mélange gazeux selon la différence de pression entre ladite détection de premier débit de gaz ou de mélange gazeux et ladite détection de second débit de gaz ou de mélange gazeux,
et **en ce que** ledit premier moyen (3, 18) pour la détection d'un premier débit de gaz ou de mélange gazeux dudit groupe de prélèvement (101) comprend un groupe de compteur-échangeur de chaleur (3) pour la mesure dudit premier débit de gaz ou de mélange gazeux et pour le refroidissement du gaz ou du mélange gazeux.

2. Système de commande et d'analyse de gaz selon la revendication 1, **caractérisé en ce que** ledit moyen (7, 8, 12) pour l'alimentation en air et le mélange de celui-ci comprend un système pneumatique (12) et un éjecteur de Venturi (8), ledit groupe de mesure (102) est relié au système pneumatique (12) qui est agencé pour envoyer de l'air à l'éjecteur de Venturi (8) positionné sur ladite première ligne (17) et apte à créer un débit aspirant le gaz ou le mélange gazeux présent dans ledit conduit de gaz (1) à travers le groupe de prélèvement (101).

3. Système de commande et d'analyse de gaz selon la revendication 1, **caractérisé en ce que** ledit groupe de compteur-échangeur de chaleur (3) est doté d'un diaphragme à orifice étalonné (18) pour la mesure dudit premier débit de gaz ou de mélange gazeux à travers la lecture de la valeur de pression de gaz ou de mélange gazeux.

4. Système de commande et d'analyse de gaz selon la revendication 3, **caractérisé en ce que** ledit groupe de compteur-échangeur de chaleur (3) comprend au moins une plaque de compteur-échangeur de chaleur (14) réalisée dans un seul corps et incorporant ledit diaphragme à orifice étalonné (18).

5. Système de commande et d'analyse de gaz selon la revendication 4, **caractérisé en ce que** ladite plaque de compteur-échangeur de chaleur (14) comprend un premier canal d'entrée (19') raccordé à ladite première ligne (17) et comprenant ledit diaphragme à orifice étalonné (18), et un second canal de retour (20') raccordé à ladite ligne de retour (17') et raccordé audit conduit de gaz (1).

6. Système de commande et d'analyse de gaz selon la revendication 5, **caractérisé en ce que** ladite plaque (14) comprend sur un côté des première et seconde voies de gaz (19, 20) et sur l'autre côté un nervurage d'échange de chaleur (21), ledit groupe de prélèvement (101) comprend en outre une plaque de couvercle (15) positionnée sur le côté desdites première et seconde voies de gaz (19, 20).

7. Système de commande et d'analyse de gaz selon la revendication 6, **caractérisé en ce que** ledit groupe de prélèvement (101) comprend une couche de matériau isolant (16) positionnée sur ladite plaque de couvercle (15) et apte à isoler thermiquement la plaque de couvercle (15) dudit conduit de gaz (1) à température élevée.

8. Système de commande et d'analyse de gaz selon la revendication 4, **caractérisé en ce que** ladite plaque (14) comprend un filtre d'aspiration (2) raccordé audit conduit de gaz (1).

9. Système de commande et d'analyse de gaz selon la revendication 1, **caractérisé en ce que** ledit second moyen (6) pour la détection d'un second débit de gaz ou de mélange gazeux comprend un diaphragme étalonné positionné en aval dudit analyseur de gaz à infrarouge (5) du groupe de mesure (102).

10. Système de commande et d'analyse de gaz selon la revendication 1, **caractérisé en ce que** ledit moyen (7, 8, 12) pour l'alimentation et le mélange d'air comprend une valve proportionnelle (7) ajustable selon la différence de pression entre la lecture dudit second débit de gaz ou de mélange gazeux par ledit second moyen (6) situé dans le groupe de mesure (102) et la lecture dudit premier débit de gaz ou de mélange gazeux par ledit premier moyen (3, 18) situé dans le groupe de prélèvement (101).

11. Système de commande et d'analyse de gaz selon la revendication 1, **caractérisé en ce que** ledit analyseur de gaz à infrarouge (5) est raccordable électriquement à un système d'actionnement de rouleau d'impression (9) d'un groupe d'impression.
